# EUROPÄISCHE PATENTSCHRIFT

(11) **EP 2 228 067 B1**
(45) Veröffentlichungstag und Bekanntmachung des Hinweises auf die Patenterteilung: **11.12.2013**
(21) Anmeldenummer: 10154281.9
(22) Anmeldetag: 22.02.2010
(51) Int. Cl.: A61K 35/74, A61P 37/08

(54) **Probiotische Zusammensetzung und deren Verwendung**
Probiotic compound and use of same
Composition probiotique et son utilisation

(43) Veröffentlichungstag der Anmeldung: 15.09.2010
(73) Patentinhaber: LR Health & Beauty Systems GmbH, 59227 Ahlen (DE)
(72) Erfinder: Larsen-Vefring, Sabine, 14089 Berlin (DE)
(74) Vertreter: Morawski, Birgit

(56) Entgegenhaltungen:
- WO-A1-03/013558
- WO-A1-2005/047489
- WO-A1-2005/060937
- WO-A2-2008/031438
- US-A1- 2008 206 171

## Beschreibung

Die vorliegende Erfindung betrifft eine Zusammensetzung aus probiotischen Mikroorganismen und deren Verwendung gemäß der Ansprüche.

Probiotika sind gemäß einer 2001 veröffentlichten Definition einer Expertenkommission zur Anwendung von Milchsäurebakterien der FAO (Food and Agriculture Organisation) und der WHO (World Health Organisation) "lebende Mikroorganismen, die, wenn in ausreichender Menge verabreicht, dem Wirtsorganismus einen gesundheitlichen Nutzen bringen". Gemäß einer weiteren Definition der LABIP (Lactic Acid Bacteria Industrial Platform) sind Probiotika "...lebende Organismen, die, wenn in bestimmten Mengen eingenommen, einen gesundheitlichen Nutzen bewirken, der über den des inhärent vorhandenen Nährwertes hinausgeht....".

Im Lebensmittelbereich werden überwiegend Milchsäurebakterien, vor allem Lactobacillen und Bifidusbakterien verarbeitet, im arzneilichen Bereich u.a. auch apathogene Kolibakterien, Enterokokken und Hefen.

"Prebiotika" oder "Präbiotika" sind Nährstoffe für die mikroökologisch erwünschten Mikroorganismen im Dickdarm und fördern bei oraler Verabreichung das Wachstum dieser Keime und unterstützen die Bildung der "Säuerungsflora" im Dickdarm. Gleichzeitig sollen Präbiotika das Wachstum unerwünschter Keime, die sogenannte "Fäulnisflora" unterdrücken. Es handelt sich dabei um komplexe Kohlenhydrate, die im menschlichen Dünndarm nicht gespalten werden können und daher im Dickdarm dem Stoffwechsel der Darmflora zur Verfügung stehen. Während herkömmliche Ballaststoffe generell das Mikrobenwachstum im Kolon stimulieren, fördern Prebiotika besonders die Vermehrung von Milchsäurebakterien wie Bifidusbakterien und Lactobacillen.

Die Stellungnahme "Fructooligosaccharide und Inulin" der Arbeitsgruppe "Fragen der Ernährung" der lebensmittelchemischen Gesellschaft aus dem Jahr 2002 beschreibt, dass in Humanstudien zur Wirkung von Prebiotika die bifidogene Wirkung von löslichen Ballaststoffquellen aus Fructooligosacchariden und Inulin belegt werden konnte. Prebiotika fördern sowohl beim Erwachsenen als auch beim Säugling die Vermehrung von Bifidusbakterien im Darm und bewirken eine gesundheitsförderliche Verschiebung in der Zusammensetzung der Dickdarmflora.

Werden Pre- und Probiotika in einem Präparat vereinigt, spricht man von Synbiotika. Diese bestehen aus probiotischen Stämmen und prebiotischen Kohlenhydratpolymeren, z.B. Fructooligosaccharide (FOS).

Die Vorstellung, dass probiotische Stämme allergische Symptome verzögern oder verhindern können, beruht auf der sogenannten "Hygiene-Hypothese" (Vercelli, Curr. Opin. Immunol. 2006; 18: 733-7). Diese Hypothese beruht darauf, dass in den letzten Jahrzehnten in den industrialisierten Ländern ein Rückgang von Infektionskrankheiten verzeichnet wird bei gleichzeitigem Anstieg von Allergien. Bei zunehmender Hygiene in den Haushalten werde das Immunsystem von Säuglingen nicht mehr ausreichend entwickelt und führe zu einer Dysbalance zwischen verschiedenen Subtypen regulatorischer T-Lymphozyten, den sog. T-Helfer-1-Zellen (TH₁-Zellen) und den T-Helfer-2-Zellen (TH2-Zellen, Schulze et al, Probiotika Mikroökologie, Mikrobiologie, Qualität, Sicherheit und gesundheitliche Effekte, Hippokrates Verlag Stuttgart, 2008). Dieses Ungleichgewicht zugunsten der TH₂-Zellen im Immunsystem ist typisch für Allergiker. Durch Verabreichung von Probiotika bei Säuglingen soll die Proliferation von TH₂-Zellen reduziert werden, so dass die TH₁-Zellen dominieren können. So wird das Immunsystem auf Infektabwehr trainiert.

Bei Patienten mit Allergien konnte gezeigt werden, dass sowohl die Darmimmunologie als auch die intestinale Mikroökologie betroffen sind. Die natürliche Barrierefunktion der Darmschleimhaut ist gestört. Dadurch wird die Darmschleimhaut durchlässig für großmolekulare, allergene Nahrungsbestandteile und Mikroorganismen. Insofern befassen sich Allergikertherapien mit der Behandlung der gestörten Immunitätslage, der Dysbalance der Mikroökologie im Darm und der Wiederherstellung einer gewöhnlichen Darmpermeabilität.

Kalliomäki et al. (Lancet 2001, 357:1076-1057) untersuchten in einer randomisierten, placebokontrollierten, klinischen Doppelblind-Studie den Einfluss von *Lactobacillus rhamnosus* GG (LGG) an genetisch vorbelasteten Kindern vor der Entwicklung von Neurodermitis (atopisches Ekzem) bei einer täglichen Verabreichung von 2x10¹⁰ KBE (Kolonie bildenden Einheiten). 159 Schwangere wurden zwei bis vier Wochen vor der Entbindung in die Studie aufgenommen. Voraussetzung war, dass sie selbst oder ihr Partner an Neurodermitis, allergischer Rhinitis oder Asthma erkrankt waren oder einen entsprechenden familiären Hintergrund aufwiesen. 82 Schwangere erhielten ein Placebo, 77 Schwangere das Laktobazillenpräparat. Nach der Geburt erhielten die stillenden Mütter für weitere 6 Monate die Studienselbstmedikation. Im Falle des Abstillens erhielten die Säuglinge bis zum Alter von 6 Monaten das Placebo oder LGG direkt. Die Kinder wurden über 2 Jahre beobachtet, gefolgt von einem follow-up über weitere 2 Jahre (Kalliomäki et al. Lancet 2003, 361:1869-1871). 68 Kinder der Placebo- und 64 Kinder der LGG-Gruppe beendeten nach 2 Jahren die Studie. Zum follow-up nach 4 Jahren standen noch 54 (Placebo) und 53 Kinder (LGG) zur Verfügung. Zu beiden Zeitpunkten waren im Vergleich zur Kontrollgruppe bis zu 50% weniger Kinder an Neurodermitis erkrankt, nach zwei Jahren 46% vs 23%, nach vier Jahren 46% vs 26%. Zahlreiche weitere klinische Studien folgten. Insgesamt ist feststellbar, dass die Wirksamkeit einer Bakterienart nicht, ohne es geprüft zu haben, auf eine andere, selbst innerhalb der gleichen Gattung, übertragen werden darf.

In zahlreichen Veröffentlichungen wird der Nutzen von Probiotika auch im Hinblick auf Allergien dargelegt. So wird in einem Übersichtsartikel von Baumbach (OM & Ernährung, 2008, 123:F9-F15) auf den therapeutischen Nutzen von Probiotika hingewiesen und Therapiererfolge bei atopischer Dermatitis beschrieben. Insbesondere ist eine positive Wirkung von *L. rhamnosus* bei allergischen Reaktionen auf Kuhmilchproteine feststellbar.

Auch Parvez et al. (J. Appl. Microbiol. 2006: 100 1171-1185) befassen sich mit der Wirkung von Probiotika auf Allergien und Ekzeme. Des Weiteren beschreibt Elizabeth Furrie (Proceedings of Nutrition Society, 2005, 64, 465-469) die Anwendung von *Lactobacillus rhamnosus* bei Asthma, Rhinitis, Ekzemen und Lebensmittelallergien, *Bifidus lactis* bei atopischen Ekzemen, *Lactobacillus casei* bei allergischer Rhinitis (Heuschnupfen) und *Lactobacillus reuterii* bei atopischer Dermatitis. Therapeutische Effekte von probiotischen Produkten bei Patienten mit Neurodermitis werden von Yim et al. beschrieben (J. Microbiol. Biotechnol., 2006, 16: 1699-1705). Die Wirkung von *Lactobacillus paracasei* bei Lactoseintoleranz wurde ebenfalls nachgewiesen von (Gaon et al. Medicina Buenos Aires, 1995, 55: 237-242)

Zusammenfassend ist zu sagen, dass Probiotika über ein breit angelegtes, aber individuell unterschiedliches Wirkungsspektrum verfügen, das durch Interaktion sowohl mit der wirtseigenen Mikroflora als auch mit dem Wirtsorganismus selbst geprägt ist. Neben antimikrobiellen Wirkungen, Wirkungen auf intestinale und extraintestinale Ökosysteme, Wirkungen auf die Barrierefunktion des Darmepithels, auf das Immunsystem, antiarthritische und antikarzinogene Wirkungen sowie Wirkungen auf den Lipidstoffwechsel, sind Probiotika auch zur Vorbeugung und Behandlung von allergischen Reaktionen einsetzbar.

Die Verwendung und Wirksamkeit von Probiotika einzeln oder in Kombination auch mit anderen Mikroorganismen ist seit langem bekannt.

So wird in der EP 1 773 161 B1 eine Zusammensetzung aus drei probiotischen Mikroorganismen *Bifidobakterium lactis, Lactobacillus casei subsp. Rhamnosus* und *Lactobacillus plantarum* und deren Wirksamkeit bei der Behandlung von Atemwegsinfektionen oder Darmträgheit beschrieben.

Aus der EP 888 11B B1 ist eine probiotische Zusammensetzung umfassend ein oder mehrere probiotische Mikroorganismen, Stärke und Oligosaccharide bekannt, die eine längere Lebenszeit der Mikroorganismen im Darmtrakt ermöglicht.

Als ein weiteres Beispiel sei auf die DE 60 2005 001 892 T2 hingewiesen, in welcher eine Lebensmittelzusammensetzung umfassend mindestens zwölf Stämme an probiotischen Bakterien, präbiotischen Substanzen, Aloe und Carrageenans beschrieben ist, wobei dem Dokument keine konkrete Auflistung von zur Verwendung geeigneten probiotischen Stämmen entnehmbar ist.

Ein bisher nur unzureichend gelöstes Problem bei der Verabreichung von probiotischen Zusammensetzungen besteht allgemein in der pH-Instabilität der Mikroorganismen, so dass diese unter den stark aciden Bedingungen des menschlichen Verdauungstraktes im Magen und der Galle nicht lebensfähig sind und daher abgetötet werden. Aus diesem Grund erreicht letztendlich nur ein kleiner Teil der Bakterien den Darmtrakt, so dass die Wirksamkeit der Probiotika stark eingeschränkt ist.

Eine Aufgabe der vorliegenden Erfindung ist daher die Bereitstellung einer probiotischen Zusammensetzung, die sowohl über einen langen Zeitraum stabil gelagert werden kann und eine erhöhte pH-Stabilität gegenüber Säuren, wie Magen- oder Gallensäuren aufweist.

Diese Aufgabe wird erfindungsgemäß durch eine Zusammensetzung mit den Merkmalen des Anspruchs 1 gelöst.

Demnach umfasst die Zusammensetzung mindestens zwölf probiotische Mikroorganismen wobei die Zusammensetzung mindestens 6 Lactobacillusstämme, mindestens 5 Bifidusbakterienstämme und mindestens einen Stamm aus der Gruppe der Streptokokken umfasst.

Ein wesentlicher Vorteil der erfindungsgemäßen Zusammensetzung besteht in deren besonderen Stabilität gegenüber einer sauren Umgebung so dass die Probiotika den Darmtrakt durch den Magen unbeschadet, in lebender Form erreichen, den Darm besiedeln und ihre Wirksamkeit insbesondere auf den menschlichen Organismus entfalten können.

In einer bevorzugten Ausführungsform sind die probiotischen Mikroorganismen ausgewählt aus einer Gruppe enthaltend *Lactobacillus acidophilus* LH5, *Lactobacillus casei* LC1, *Lactobacillus plantarum* LP1, *Lactobacillus rhamnosus* LR3, *Lactobacillus paracasei* LPC, *Lactobacillus bulgaricus* LG1, *Bifidus longum* BG3, *Bifidus breve* BR2, *Bifidus bifidum* BF2, *Bifidus lactis* BL2, *Bifidus infantis* BT1 und *Streptococcus thermophilus* ST3. Weitere probiotische Mikroorganismen, die Verwendung finden können, sind *Lactobacillus buchneri, L. brevis, L. cellobiosus, L. collinoides, L. coprophillus, L. crispatus, L. curvatus, L. delbrückkii ssp. delbrückkii, L. delbrückkii ssp. bulgaricus, L. delbrückkii ssp.lactis, L.fermentum, L.fructiforans, L. gasseri, L.helveticus, L. johnsonii, L. lindnerl, L. reuterii, L.periosus, L.salivarius, Lactococcus lactis SL6, Bifidus faecium, Bifidus faecalis, Bifidus adolescens,* und *Bifidus animalis*

Die aufgelisteten Stämme sind für ihre antiallergene Wirkung bekannt. Im Rahmen der Untersuchungen, die zur Entwicklung der vorliegenden Zusammensetzung führten, konnte neben einer erhöhten Stabilität nicht nur eine Addition des antiallergenen Effektes, sondern überraschenderweise ein Synergismus der antiallergenen Wirkungsweise nachgewiesen werden. Die erfindungsgemäße Zusammensetzung ist somit insbesondere zur Vorbeugung und Behandlung von allergischen Reaktionen bei Mensch und Tier, insbesondere von Neurodermitis, Asthma oder und/oder Rhinitis geeignet und kann bevorzugt in Form eines Nahrungsergänzungsmittels oder auch eines Medikamentes verwendet werden.

Es ist weiterhin von Vorteil, wenn die probiotischen Mikroorganismen mit mindestens einem Protein und mindestens einem Polysaccharid beschichtet, insbesondere doppelt beschichtet, vorliegen.

Ein derartiges doppelschichtigen Coatingsystem ist aus der EP1514553B1 bekannt. Die erste, innere Schicht bestehend aus einer Peptid/Protein-Matrix bewirkt eine Erhöhung der pH-Stabilität der oral aufgenommenen probiotischen Bakterien und bewirkt einen zusätzlichen Schutz der Mikroorganismen unter den Bedingungen im Verdauungstrakt. So erreichen die Bakterien den Darm durch den Magen ohne durch die Magen- und Gallensäure abgetötet zu werden und können im Darm ihre physiologischen Aktivitäten ausüben.

Die zweite, äußere Schicht besteht aus einer Polysaccharid/Hydrocolloid-Matrix, die zu einer Erhöhung der Lagerstabilität führt, da lyophilisierte Milchsäurebakterien an sich äußerst sensibel gegenüber Luft, Feuchte, Temperatur sind, was eine Instabilität während der Lagerung und Verteilung sowie eine schlechte Verarbeitung zu Fertig- oder Halbfertigerzeugnissen hervorruft.

Die doppelte Beschichtung der Probiotika ermöglicht also einen Schutz vor Denaturierung und erhöht andererseits die Stabilität der Zusammensetzung.

In einer weiteren Ausführungsform umfasst die Zusammensetzung mindestens eine prebiotische Verbindung ausgewählt aus einer Gruppe enthaltend Oligosaccharide, Inulin, Pektin, Glucane, Galaktane, Dextrose, Galactooligosaccharide, Sojaoligosaccharide, Raffinosesaccharide, Stachyose, Bananenfaser, Mannane, Guarkernmehl, Konjakmehl, Xanthan, Pentosan, beta-Glucan, Arabinan, Galactan, Arabinogalactan und Mischungen aus diesen. Insbesondere ist die Verwendung von Fructooligosacchariden als Präbiotikum bevorzugt.

Der Zusammensetzung können weitere Inhaltsstoffe zugefügt werden. Beispielsweise kann die Zusammensetzung die üblichen pharmazeutischen Zusatz- und/oder Hilfsstoffe, insbesondere Füll-, Streck-, Binde-, Netz-, Stabilisierungs-, Färbe-, Puffer-, Riech-, Geschmacksstoffe enthalten.

So kann die Zusammensetzung mindestens einen Füllstoff, mindestens einen Trägerstoff und mindestens ein Trennmittel umfassen.

Füllstoffe sind Stoffe ohne oder fast ohne Nährwert, die durch Volumenerhöhung eines Lebensmittels bzw. Nahrungsergänzungsmittels den Nährstoff-, insbesondere den Fettgehalt, gezielt verdünnen, diesem Textur und Körper verleihen, vom Menschen nur geringfügig oder gar nicht verdaut oder resorbiert werden, sowie zusätzlich die Resorptionsrate und -geschwindigkeit anderer Nährstoffe verringern können.

Der mindestens eine Füllstoff ist dabei vorteilhafterweise ausgewählt aus einer Gruppe enthaltend Cellulose, Lactose, Saccharose, Amylose, Dextrose, Mannit, Inosit, Maltodextrin, Maisstärke, Reisstärke, Ballaststoffe, Calciumhydrogenphosphate und Hydrokolloide.

Trennmittel dagegen werden eingesetzt zur Verbesserung oder Erhaltung der Streu- und Rieselfähigkeit von Pulvern. Das mindestens eine Trennmittel ist bevorzugt ausgewählt aus einer Gruppe enthaltend Talkum, Magnesium- oder Calciumcarbonat, Cellulose bzw. mikrokristalline Cellulose, Magnesiumcarbonat, Magnesiumsalze von Speisefettsäuren, kolloidale Kieselsäure, Siliciumdioxid, Natrium-, Magnesium-, Calcium- und Aluminiumsilikate, Bentonit, Dicalciumdiphosphat, Calciumnatriumpolyphoshat, Calciumpolyphosphate und Stearinsäure.

Trägerstoffe sind feste Zusätze zu Lebensmitteln anorganischer oder organischer Natur, die lediglich der Aufnahme, Fixierung oder besseren Handhabung wie z.B. Dosiererleichterung, zur Schutzfunktion oder als Schutzkolloid anderer Stoffe dienen. Der mindestens eine Trägerstoff ist bevorzugt ausgewählt aus einer Gruppe enthaltend Lecithine, Alginate, Traganth, Gummi arabicum, Glucitol, Pektine, Carboxymethylcellulose, Saccharose, Milchzucker und Stärke.

Als Streckstoffe können z.B. Lactose, Dextrose, Saccharose, Cellulose, Maisst-arke oder Kartoffelst·arke verwendet werden, Typische Gleitmittel sind Silicat, Talk, Stearinsäure, Magnesium- oder Calciumstearat und/oder Polyethylenglykole. Als Bindemittel finden Stärken, Gummi arabicum, Gelatine, Methylcellulose, Carboxymethylcellulose oder Polyvinylpyrrolidon Verwendung. Typische Benetzungsmittel sind Lecithin, Polysorbate, Laurylsulfate. Stabilisierungsmittel umfassen u.a. Polyole, Aminosäuren, Polymere von Aminosäuren mit einer geladenen Seitengruppe bei physiologischem pH-Wert und Cholinsalze. Als Geschmacksstoffe können Süßungsmittel verwendet werden.

In einer Ausführungsform weist die probiotische Zusammensetzung folgende quantitative Zusammensetzung auf:
- 1 bis 20 Gew% einer Mischung aus mindestens zwölf probiotischen Mikroorganismen, bevorzugt 2 bis 15 Gew%, insbesondere bevorzugt 4 bis 10 Gew%
- 10 bis 60 Gew% mindestens einer präbiotischen Substanz, bevorzugt 20 bis 50 Gew%, insbesondere bevorzugt 30 bis 40 Gew%,
- 0 bis 70 Gew % mindestens eines Hilfsstoffes.

Als Hilfsstoffe werden bevorzugt Füllstoffe, Trennmittel und Trägerstoffe verwendet, wobei die Zusammensetzung 10 bis 70 Gew% an mindestens einem Füllstoff, bevorzugt 20 bis 60 Gew%, insbesondere bevorzugt 30 bis 55 Gew%, 0 bis 5 Gew % an mindestens einem Trennmittel, bevorzugt 1 bis 3 Gew%, und 0 bis 5 Gew % an mindestens einem Trägerstoff, bevorzugt 1 bis 3 Gew% aufweisen kann.

Die zum Einsatz kommenden probiotischen Mikroorganismen weisen typischerweise in der Zusammensetzung eine Anzahl an lebenden Zellen von mindestens 1 x 10⁹ KBE/g auf, bevorzugt von 1 x 10¹⁰ KBE/g, insbesondere bevorzugt von 1 x 10¹¹ KBE/g auf.

Die quantitative Zusammensetzung bemisst sich einerseits an der Grammatur und andererseits an der Konzentration der Mikroorganismen (KBE/g). Insofern ist neben der Grammatur, die Konzentration an koloniebildenden Einheiten bzw. Gesamtkeimzahl von wesentlicher Bedeutung. Der hohe Anteil an Präbiotika garantiert den Mikroorganismen eine ausreichend Menge an Substrat. Die Anteile an Hilfsstoffen sind technisch bedingt.

Die erfindungsgemäße Zusammensetzung weist bevorzugt eine Lagerstabilität von bis zu 18 Monaten, jedoch von mindestens 7 Wochen, bevorzugt von 9 Wochen, insbesondere bevorzugt von 11 Wochen bei Raumtemperatur (20°C) auf. Unter Lagerstabilität ist dabei die Stabilität der probiotischen Mikroorgansimen in der Zusammensetzung zu verstehen, d.h. dass die Gesamtkeimzahl der Mikroorganismen gar nicht oder nur geringfügig abnimmt. Für eine Simulation der Lagerstabilität wird die Lagertemperatur auf 37°C im Rahmen eines sogenannten Stresstestes erhöht. Bei der Lagerung bei 37°C erreicht man etwa 50% der Lagerstabilität von der bei 20°C (Raumtemperatur). Die verbesserte Stabilität der vorliegenden Zusammensetzung wurde im Vergleich zu den einzelnen doppelt beschichteten Stämmen nachgewiesen.

In einer bevorzugten Ausführungsform weist die Zusammensetzung eine verbesserte Stabilität gegenüber Säure, insbesondere Salzsäure und Gallensäure auf. So wird die Gesamtkeimzahl in Gegenwart von Salzsäure bei einer anfänglichen Gesamtkeimzahl von 3 x 10¹² innerhalb von 30 Minuten bei 37°C lediglich auf 1 x 10⁵ und innerhalb von 1 Stunde auf 1 x 10⁴ reduziert. In Gegenwart von Gallensäure erfolgt ein Abbau der Gesamtkeimzahl von 3 x 10¹² innerhalb von 30 Minuten bei 37°C auf 2 x 10⁸ und innerhalb von 1 Stunde auf 1,5 x 10⁸ reduziert.

Die Zusammensetzung liegt bevorzugt in Kapselform vor. Aber auch weitere Darreichungsformen wie Pulver oder Granulate, Dragees, Kau-, Lutsch-, Schluck- oder Schmelztabletten, Kau- oder Lutschpastillen sind möglich. Als Kapselmaterial wird bevorzugt Hydroxypropylmethylcellulose (HPMC), aber auch Gelatine wie z.B. Hartgelantine in Form von Schwein-, Rinder- oder Fischgelantine und andere herkömmliche Kapselmaterialien verwendet. Als Farbstoff kann Titandioxid verwendet werden oder andere zugelassene Farbstoffe.

Die Erfindung wird nachfolgend anhand von Ausführungsbeispielen unter Bezugnahme auf die Figuren näher erläutert.

Es zeigen
- Figur 1: ein erstes Diagramm zum Nachweis der Stabilität einer bevorzugten Zusammensetzung gemäß der vorliegenden Erfindung im Vergleich zu singulären Lactobacillen bei einer Temperatur von 37°C;
- Figur 2: ein zweites Diagramm zum Nachweis der Stabilität einer bevorzugten Zusammensetzung gemäß der vorliegenden Erfindung im Vergleich zu singulären Bifidusbakterien bei einer Temperatur von 37°C;
- Figur 3: ein drittes Diagramm zum Nachweis der Feuchteaufnahme einer bevorzugten Zusammensetzung gemäß der vorliegenden Erfindung im Vergleich zu singulären Lactobacillen und Bifidusbakterien bei einer Temperatur von 37°C;
- Figur 4: ein viertes Diagramm zum Nachweis der Stabilität einer bevorzugten Zusammensetzung in Gegenwart von Salzsäure; und
- Figur 5: ein fünftes Diagramm zum Nachweis der Stabilität einer bevorzugten Zusammensetzung in Gegenwart von Gallensäure.

### Ausführungsbeispiel 1: Zusammensetzung

Die Zusammensetzung umfasst zwölf verschiedene probiotische Stämme, ein Prebiotikum, weitere technische Hilfsmittel, das Kapselmaterial HPMC und Titandioxid als Farbstoff.

Die Rezepturkomponenten lauten wie folgt:

**Tabelle 1: Komponenten einer Zusammensetzung gemäß der vorliegenden Erfindung**

| No | Bezeichnung | Menge [1 kg] |
|---|---|---|
| 1 | *Lactobacillus acidophilus* LH5 | 4 g |
| | Gendatenbank-Nr. AY763430 | |
| | KCTC-Nr. 3925 | |
| | CBT-Nr. 1018 | |
| *2* | *Lactobacillus casei* LC1 | 4 g |
| | Gendatenbank-Nr. AY699577 | |
| | KCTC-Nr. 3930 | |
| | CBT- Nr. 1110 | |
| *3* | *Lactobacillus plantarum* LP1 | 6 g |
| | Gendatenbank-Nr. AY735404 | |
| | KCTC-Nr.3928 | |
| | CBT- Nr. 1202 | |
| *4* | *Lactobacillus rhamnosus* LR3 | 6 g |
| | Gendatenbank-Nr. AY675253 | |
| | KCTC-Nr.3929 | |
| | CBT- Nr. 1711 | |
| *5* | *Lactobacillus paracasei* LPC | 4 g |
| | Gendatenbank-Nr. AY675255 | |
| | CBT-Nr.1108 | |
| *6* | *Lactobacillus bulgaricus* LG 1 | 5 g |
| | Gendatenbank-Nr. | |
| | CBT- Nr. 1303 | |
| *7* | *Bifidus longum* BG3 | 5 g |
| | Gendatenbank- Nr. AY735403 | |
| | KCTC-Nr. 5084 | |
| | CBT- Nr. 2011 | |
| *8* | *Bifidus breve* BR2 | 4 g |
| | Gendatenbank-Nr. AY735402 | |
| | KCTC-Nr. 5081 | |
| | CBT- Nr. 2103 | |
| 9 | *Bifidus bifidum* BF2 | 4 g |
| | Gendatenbank-Nr. AY694148 | |
| | KCTC-Nr. 5082 | |
| | CBT- Nr. 2203 | |
| 10 | *Bifidus lactis* BL2 | 6 g |
| | Gendatenbank-Nr. AY700230 | |
| | CBT- Nr. 2502 | |
| 11 | *Bifidus infantis* BT1 | 4 g |
| | Gendatenbank-Nr. AY699578 | |
| | CBT-Nr. 2303 | |
| 12 | *Streptococcus thermophilus* ST3 | 6 g |
| | Gendatenbank-Nr. AY675258 | |
| | KCTC-Nr. 3927 | |
| | CBT- Nr. 3020 | |
| 13 | Fructooligosaccharide | 400 g |
| 14 | Maisstärke | 200 g |
| 15 | Maltodextrin | 220 g |
| 16 | Mikrokristalline Cellulose | 112 g |
| 17 | Siliciumdioxid | 10 g |

Herstellungsprozess: Die probiotischen Kulturen wurden mittels eines Verfahrens beschrieben in der EP 1 514 553 B1 hergestellt. Das Verfahren umfasst die Schritte Fermentation, Abtrennen der hergestellten Biomasse, Zugabe der Proteinkomponente für die erste Hülle, Zugabe des Polysaccharides für die äußere Hülle, Gefriertrocknen, Mahlen und Verpacken.

### Ausführungsbeispiel 2: Bestimmung der Mikroorganismen und Lagerstabilität

Für die mikrobiologische Untersuchung werden die Mikroorganismen aus der Doppelbeschichtung ("Dual coating") freigesetzt. Ohne diese Freisetzung (Entcoating) ist eine mikrobiologische Untersuchung und Quantifizierung nicht möglich.

Hierzu werden die Proben zur Untersuchung auf Lactobacillus und Lactococcus gegenüber den Bifidusbakterien auf unterschiedlichem Wege vorbehandelt. Die Proben werden in einem sterilisierten Puffer (aerobe Lösung für Lactobacillen, anaerobe für Bifidusbacterien) gelöst und über einen Auto-Vortex aufgeschlossen. Bei dieser Untersuchung ist explizit auf die Einhaltung der vorgesehenen Zeiten (2 Minuten schütteln, 3 Minuten warten, etc.) über eine Gesamtzeit von 20 Minuten zu achten.

Nach dieser Aufarbeitung wird mikroskopisch geprüft, ob das Decoating erfolgreich war. Andernfalls ist die Prozedur zu wiederholen. Die Bebrütung findet unter Einhaltung der erforderlichen Verdünnungsstufen mit dem vorgesehenen Puffer in MRS- für Lactobacillen und Lactococcus oder BL-Medium für Bifidusbakterien statt. Die Inkubation benötigt 48h bei 37°C für die aerobe Bebrütung auf MRS-Agar und 72h für anaerobe Bebrütung auf BL-Agar.

Eine Identifizierung erfolgt mittels API50CHL. Es handelt sich dabei um einen Identifikationstest-Kit der Firma Biomerieux zur Identifizierung von Lactobacillus und verwandten Gattungen. Der API50CHL-Streifen besteht aus 50 Mikroröhrchen zur Prüfung der Fermentation von Substraten, die zu den Kohlenhydraten und ihren Derivaten gehören. Die Fermentationsreaktionen werden mit API50CHL Medium beimpft, das die Substrate rehydriert. Während der Inkubation wird die Fermentation durch eine Farbänderung im Röhrchen angezeigt. Die anaerob gebildete Säure bewirkt einen Farbumschlag des pH-Indikators im gewählten Medium. Mit diesem System können insbesondere folgenden Mikroroganismen identifiziert werden: *Lactobacillus acidophilus, L.buchneri, L.brevis, L.cellobiosus, L.collinoides, L.coprophillus, L.crispatus, L.curvatus, L. delbrückkii ssp. delbrückkii, L.delbrückkii ssp. bulgaricus, L.delbrückkii ssp. lactis, L.fermentum, L.fructiforans, L helveticus, L lindneri, L.paracasei ssp. paracasei, L.periosus, L.plantarum, L.rhamnosus und L.salivarius.*

Im vorliegenden Ausführungsbeispiel erfolgt die Identifizierung und Quantifizierung der jeweiligen der Summe an Lactobacillen und an Bifidusbakterien ohne weitere Differenzierung.

Anhand der im Ausführungsbeispiel 1 beschriebenen Zusammensetzung wurde ein Stabilitätstest bei Lagerung der Zusammensetzung aus den zwölf doppelt beschichteten Mikroorganismen (Probiotic12) bei Raumtemperatur (20°C) und bei 37°C durchgeführt.

Für diesen Test wurden zunächst Dosen enthaltend die Zusammensetzung mit den vorgesehenen Vorsichtsmaßnahmen (Trocknungsmittel: 1,8g-Silicagel-Beutelchen und Versiegelung der Dosen bevorzugt in Form einer aluminiumkaschierten Versiegelung) sowie Dosen mit der Zusammensetzung und ohne Trocknungsmittel und Dosenversiegelung im Vergleich eingesetzt.

Die Stabilität einer Zusammensetzung aus zwölf probiotischen Stämmen (Probiotic12) wurde ohne oder in Gegenwart von Trocknungsmitteln (TM) in Form von Silicagel bei Raumtemperatur oder bei 37°C und ohne oder mit Versiegelung über einen Zeitraum von 14 Wochen getestet.

Wie der Tabelle 2 entnehmbar ist, ist die untersuchte Rezeptur in ihrer Zusammensetzung unabhängig von Trocknungsmittel und Dosenversiegelung bei Raumtemperatur gleichermaßen stabil.

Für die mikrobiologischen Untersuchungen der genommenen Proben wurden zunächst Verdünnungsstufen der Proben mit Pepton hergestellt. Die Untersuchung auf Lactobacillen erfolgt auf MRS-Agar nach Inkubation unter aeroben Bedingungen für 3 Tage bei 37°C und auf Bifidusbakterien auf BL-Agar nach Inkubation unter anaeroben Bedingungen für 5 Tage bei 30°C. Die Trockenmasse würde gemäß SOP bestimmt.

Während der Lagerung der Proben über einen Zeitraum von 14 Wochen bleibt die Konzentration an Bifidusbakterien über den gesamten Zeitraum bei Raumtemperatur konstant (2x10¹⁰KBE/g bis 1x10¹⁰KBE/g), während bei einer Lagerung bei 37°C die Keimkonzentration an Bifidusbakterien geringfügig abnimmt (siehe Tabelle 2)

Im Gegensatz dazu nimmt die Konzentration an Lactobacillen und Lactococcen um den Faktor 10 nach Lagerung der Zusammensetzung bei 37°C ab, wohin hingegen die Konzentration an Lactobacillen und Lactococcen während der Lagerung bei Raumtemperatur über einen Zeitraum von 11 Wochen relativ konstant (12x10⁹KBE/g bis 5x10⁹KBE/g) bleibt (Tabelle 2).

Die einzelnen Ergebnisse sind der nachfolgenden Tabelle 2 zu entnehmen.

**Tabelle 2: Ergebnisse der Lagerstabilität der Zusammensetzung aus 12 probiotischen Mikroorganismen mit Trocknungsmitteln und Versiegelung und ohne Trocknungsmittel und Versiegelung**

| **Probiotische Zusammensetzung aus 12 Stämmen** | **Lagerdauer** | **Raumtemperatur** | | | **Lagerung bei 37°C** | | |
|---|---|---|---|---|---|---|---|
| | | Lactobacillen [KBE/g] | Bifidusbakterien [KBE/g] | Feuchte | Lactobacillen [KBE/g] | Bifidusbakterien [KBE/g] | Feuchte |
| mit TM und Versiegelung | 0 | 5,7x10⁹ | 1,9x10¹⁰ | k.U. | 5,7x10⁹ | 1,9x10¹⁰ | k.U. |
| | 1 Woche | 7,9x10⁹ | 1,7x10¹⁰ | 2,21 | 3,3x10⁹ | 8,7x10⁹ | 2,23 |
| | 2 Wochen | 6,5x10⁹ | 1,0x10¹⁰ | 2,93 | 3,5x10⁹ | 6,1x10⁹ | 3,17 |
| | 3 Wochen | 6,2x10⁹ | 1,7x10¹⁰ | 2.88 | 1,5x10⁹ | 3,4x10⁹ | 2.85 |
| | 4 Wochen | 9,0x10⁹ | 1,7x10¹⁰ | 3,12 | 1,0x10⁹ | 5,3x10⁹ | 3,01 |
| | 5 Wochen | 4,7x10⁹ | 1,4x10¹⁰ | 2,98 | 0,8x10⁹ | 3,5x10⁹ | 2,88 |
| | 6 Wochen | 8,4x10⁹ | 0,9x10¹⁰ | 3,09 | 1,6x10⁹ | 6,0x10⁹ | 3,03 |
| | 7 Wochen | 9,3x10⁹ | 1,5x10¹⁰ | 3,08 | 3,2x10⁹ | 7,2x10⁹ | 2,7 |
| | 11 Wochen | 1,2x10⁹ | 2,6x10¹⁰ | 3,04 | 0,5x10⁹ | 7,0x10⁹ | 2,99 |
| | 14 Wochen | 8,8x10⁹ | 1,6x10¹⁰ | 3,23 | 0,5x10⁹ | 3,0x10⁹ | 3,05 |
| ohne TM und ohne Versiegelung | 0 | 6,9x10⁹ | 1,3x10¹⁰ | 2,79 | 6,9x10⁹ | 1,3x10¹⁰ | k.U. |
| | 1 Woche | 6,1x10⁹ | 1,5x10¹⁰ | 2,84 | 4,1x10⁹ | 8,0x10⁹ | 3,0 |
| | 2 Wochen | 6,4x10⁹ | 1,5x10¹⁰ | 2,97 | 2,8x10⁹ | 8,5x10⁹ | 2,95 |
| | 3 Wochen | 6,8x10⁹ | 1,5x10¹⁰ | 2.92 | 7,1x10⁹ | 7,0x10⁹ | 2.83 |
| | 4 Wochen | 4,0x10⁹ | 1,0x10¹⁰ | 2,80 | 1,0x10⁹ | 7,6x10⁹ | 2,65 |
| | 8 Wochen | 8,3x10⁹ | 1,6x10¹⁰ | 3,04 | 0,5x10⁹ | 7,0x10⁹ | 2,97 |
| | 11 Wochen | 7,3x10⁹ | 1,4x10¹⁰ | 3,08 | 0,5x10⁹ | 6,0x10⁹ | 3,09 |

Die vorliegende Zusammensetzung ist auch stabiler bezüglich Lagerung bei Raumtemperatur und bei 37°C als die reinen, dual gecoateten Stammkulturen.

Hierzu werden die entsprechenden Proben als Pulver offen in einer Klimakammer bei 37°C gelagert, wobei Temperatur und Feuchte per Datenlogger aufgezeichnet werden. Die Lagerung jedes Versuchsansatzes erfolgt über einen Zeitraum von 1 Stunde, 5 Stunden, 24 Stunden und 48 Stunden. Nach Probenentnahme wird jeweils eine Lösung von 1g in 10ml Pepton hergestellt, die entsprechend verdünnt wird und deren Gesamtkeimzahl bestimmt wird. Die Untersuchung auf Lactobacillen erfolgt auf MRS-Agar nach Inkubation unter aeroben Bedingungen für 3 Tage bei 37°C und auf Bifidusbakterien auf BL-Agar nach Inkubation unter anaeroben Bedingungen für 5 Tage bei 30°C.

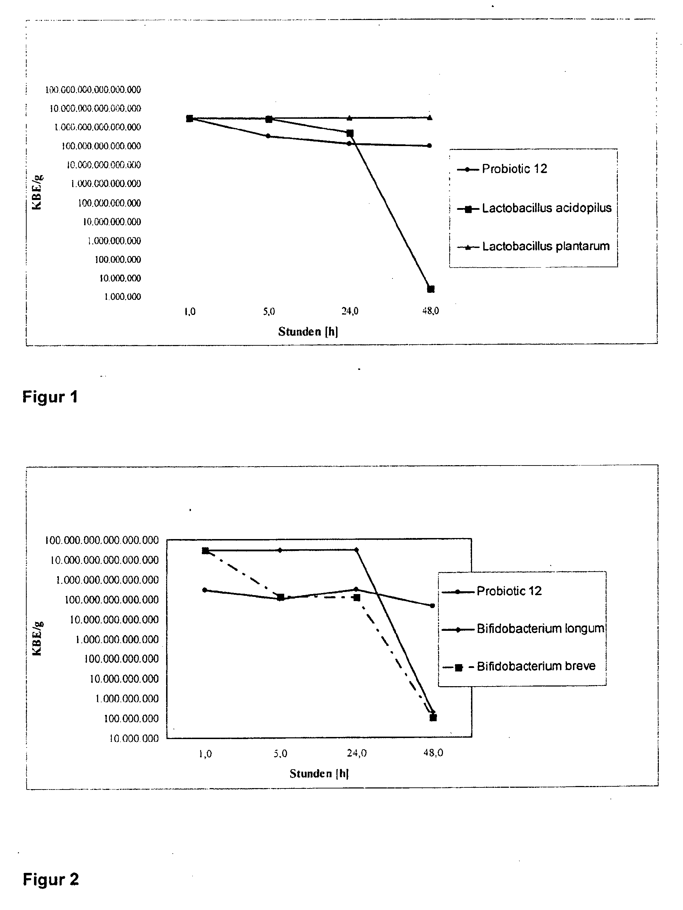

Wie aus dem Diagramm der Figur 1 entnehmbar ist, ist die Zusammensetzung aus den zwölf Stämmen in Probiotic 12 über den gemessenen Zeitraum von 4 Stunden stabil, während insbesondere der einzeln vorliegende, gecoatete Stamm *Lactobacillus acidophilus* bereits innerhalb von 4 Stunden inaktiviert wird.

Ein vergleichbares Bild ergibt sich für die getesteten Bifidobakterien (Figur 2). Sowohl *Bifidobacterium longum* als auch *Bifidobacterium breve* zeigen nach 4 Stunden einen rapiden Aktivitätsverlust, während die Zusammensetzung der zwölf Mikroorganismen, die die getesteten Bifidobakterien umfasst, auch nach 4 Stunden noch ausreichend stabil ist.

Die experimentellen Daten belegen eindeutig, dass die Stabilität der Mikroorganismen, insbesondere deren Lagerstabilität bei 37°C, in der Zusammensetzung Probiotic 12 im Vergleich zu den einzelnen Mikroorganismen wie Lactobacillus acidophilus, *Bifidobacterium longum* und *Bifidobacterium breve* deutlich und in synergistischer Weise erhöht ist.

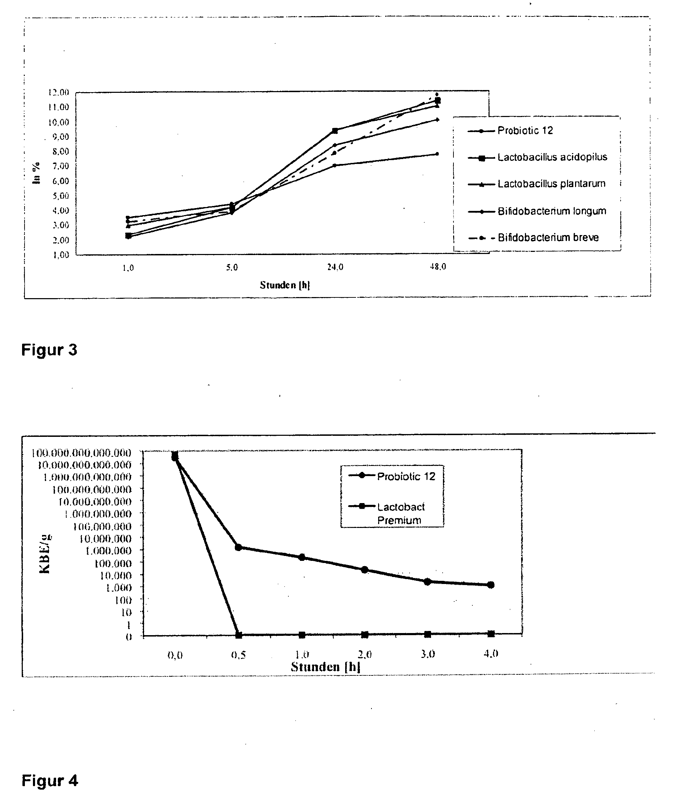

Figur 3 zeigt ein Diagramm mit experimentellen Ergebnissen zur Feuchtebeständigkeit der einzelnen Stämme und der Zusammensetzung Probiotic 12, wobei erkennbar ist, dass die einzelnen Stämme in einem höheren Maße Feuchtigkeit aufnehmen als die Zusammensetzung aus den zwölf Mikroorganismen.

### Ausführungsbeispiel 3: Säurestabilität

Die im Folgenden beschriebenen Untersuchungen befassen sich mit der Resistenz von probiotischen lyophilisierten Mikroorganismen gegenüber den Verdauungssäften des Menschen durch Behandlung mit Salzsäure zur Simulation der Magensäure und Gallensäure zur Simulation der Bedingungen des Verdauungstraktes.

Verglichen wird die Stabilität einer Zusammensetzung aus zwölf probiotischen Mikroorganismen (Probiotic12) gemäß dem Ausführungsbeispiel 1 und eine Zusammensetzung aus vier verschiedenen Bifidusbakterienstämmen, 4 verschiedenen Lactobacillusstämmen und *Streptococcus thermophilus,* das im Handel unter dem Namen Lactobact Premium erhältlich ist. Als Präbiotika sind darin Inulin und FOS enthalten, angereichert mit Kaliumchlorid, Magnesiumsulfat, Enzymen und Mangansulfat.

Zur Durchführung der Vergleichsexperimente unter Verwendung mit Salzsäure zur Simulation des Magenmilieus werden zunächst 1 g der zu testenden Zusammensetzung mit 10 ml Salzsäure (pH 1,5) vermischt. Die Mischung wird bei 37°C gelagert. Es werden in bestimmten Zeitabständen Proben gezogen und die Gesamtkeimzahl in der Probe nach geeigneter Verdünnung mit Pepton mittels Inkubation unter aeroben Bedingungen auf PC-Agar bei 30°C für 3 Tage bestimmt. Die Ergebnisse sind in Tabelle 3 und Figur 4 dargestellt.

**Tabelle 3: Gesamtkeimzahl an probiotischen Bakterien nach Inkubation mit Salzsäure zu verschiedenen Zeitpunkten**

| Zeitraum | Probiotische Zusammensetzung aus 12 Stämmen [KBE/g] | Lactobact [KBE/g] |
|---|---|---|
| Unbehandelt | > 300 x 10¹⁰ | 50 x 10¹⁰ |
| nach 30 Min. bei 37°C | 1,5 x 10⁵ | 100 |
| nach 1 Std. bei 37°C | 2,2 x 10⁴ | < 100 |
| nach 2 Std. bei 37°C | 2,0 x 10³ | < 100 |
| nach 3 Std. bei 37°C | 2000 | < 100 |
| nach 4 Std. bei 37°C | < 100 | < 100 |

Die Gesamtkeimzahl der erfindungsgemäßen Zusammensetzung aus zwölf probiotischen Bakterien (Probiotic12) beträgt bei Beginn des Versuches >3x10¹²KBE/g. Bei einer Verweildauer von ca. 30 Minuten unter den simulierten Magenbedingungen ist die Gesamtkeimzahl reduziert auf 1,5x10⁵KBE/g und fällt dann langsam ab auf 200KBE/g nach 3 Stunden.

Lactobact Premium dagegen beinhaltet eine Gesamtkeimzahl von 5x10¹¹KBE/g zu Beginn des Testes. Die Gesamtkeimzahl wird durch die Anwesenheit von Salzsäure bereits innerhalb der ersten 30 Minuten bis zur Nachweisgrenze von <100KBE/g reduziert.

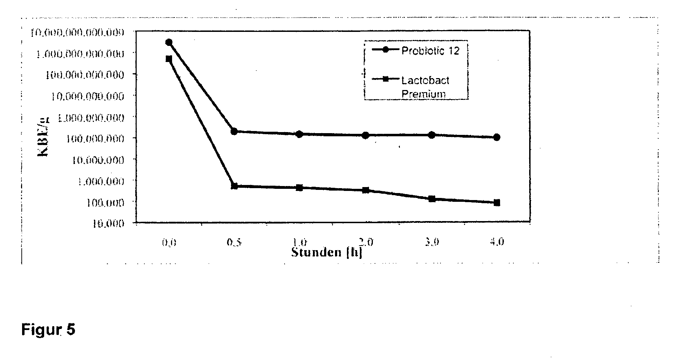

Zur Durchführung der Vergleichsexperimente unter Verwendung mit sterilfiltrierter Ochsengalle zur Simulation des Gallenmilieus werden zunächst 1 g der zu testenden Zusammensetzung mit 10 ml Ochsengalle (50 g gelöst in 100 ml dest. Wasser, pH = 7) vermischt. Die Mischung wird bei 37°C gelagert. Es werden in bestimmten Zeitabständen Proben gezogen und die Gesamtkeimzahl in der Probe nach geeigneter Verdünnung mit Pepton mittels Inkubation unter aeroben Bedingungen auf PC-Agar bei 30°C für 3 Tage bestimmt. Die Ergebnisse sind in Tabelle 4 und Figur 5 dargestellt.

**Tabelle 4: Gesamtkeimzahl an probiotischen Bakterien nach Inkubation mit Ochsengalle zu verschiedenen Zeitpunkten**

| Zeitraum | Probiotische Zusammensetzung aus 12 Stämmen [KBE/g] | Lactobact [KBE/g] |
|---|---|---|
| Unbehandelt | > 3 x 10¹² | 5 x 10¹¹ |
| nach 30 Min. bei 37°C | 2,0 x 10⁸ | 52 x 10⁴ |
| nach 1 Std. bei 37°C | 1,5 x 10⁸ | 44 x 10⁴ |
| nach 2 Std. bei 37°C | 1,3 x 10⁸ | 32 x 10⁴ |
| nach 3 Std. bei 37°C | 1,3 x 10⁸ | 12 x 10⁴ |
| nach 4 Std. bei 37°C | 1,0 x 10⁸ | 8 x 10⁴ |

Die anfängliche Gesamtkeimzahl beträgt in der Zusammensetzung gemäß Ausführungsbeispiel 1 (Probiotic12) >3x10¹²KBE/g. Bei einer Verweildauer von ca. 30 Minuten im Verdauungstrakt (simuliert durch Ochsengalle) ist die Gesamtkeimzahl reduziert auf 1-2x10⁸KBE/g und bleibt als solche stabil über den Beobachtungszeitraum von 0,5 bis 4 Stunden.

Lactobact Premium dagegen beinhaltet eine Gesamtkeimzahl von 5x10¹¹KBE/g zu Beginn des Testes. Die Gesamtkeimzahl wird durch die Anwesenheit von Gallensäure innerhalb der ersten 30 Minuten reduziert von 5x10¹¹KBE/g auf 5,2x10⁵KBE/g und nimmt dann stetig ab über den Versuchszeitraum bis zu 4 Stunden.

Bei einem Vergleich der experimentellen Ergebnisse ist somit zu sagen, dass die vorliegende Zusammensetzung aus zwölf probiotischen Mikroorganismen (Probiotic12) aufgrund seiner quantitativen und qualitativen Zusammensetzung und der Qualität der verwendeten probiotischen Stämme stabiler gegen Salzsäure (pH 1,5) und gegen Gallensäure (gesättigt und sterilfiltriert) ist als ein vergleichbares Handelsprodukt.

## Patentansprüche

1. Zusammensetzung umfassend mindestens zwölf probiotische Mikroorganismen, wobei die Zusammensetzung mindestens 6 Lactobacillusstämme, mindestens 5 Bifidusbakterienstämme und mindestens einen Stamm aus der Gruppe der Streptokokken umfasst.

2. Zusammensetzung nach Anspruch 1, **dadurch gekennzeichnet, dass** die Mikroorganismen ausgewählt sind aus einer Gruppe enthaltend *Lactobacillus acidophilus* LH5, *Lactobacillus casei* LC1, *Lactobacillus plantarum* LP1, *Lactobacillus rhamnosus* LR3, *Lactococcus lactis* SL6, *Lactobacillus paracasei* LPC, *Lactobacillus bulgaricus* LG1, *Bifidus longum* BG3, *Bifidus breve* BR2, *Bifidus bifidum* BF2, *Bifidus lactis* BL2, *Bifidus faecium, Bifidus faecalis, Bifidobacterium infantis* BT1, *Streptococcus thermophilus* ST3, *Lactobacillusbuchneri, L.brevis, L.cellobiosus, L.collinoides, L.coprophillus, L.crispatus, L.curvatus, L.delbrückkii ssp. delbrückkii, L.delbrückkii ssp. bulgaricus, L.delbrückkii ssp. lactis, L.fermentum, L.fructiforans, L. gasseri, L. helveticus, L. johnsonii L.lindneri, ,L. reuterii, L. periosus, L. salivarius, Bifidus adolescens* und *B. animalis.*

3. Zusammensetzung nach Anspruch 1 oder 2, **dadurch gekennzeichnet, dass** die Mikroorganismen ausgewählt sind aus einer Gruppe enthaltend *Lactobacillus acidophilus* LH5, *Lactobacillus casei* LC1, *Lactobacillus plantarum* LP1, *Lactobacillus rhamnosus* LR3, *Lactobacillus paracasei* LPC, *Lactobacillus bulgaricus* LG1, *Bifidus longum* BG3, *Bifidus breve* BR2, *Bifidus bifidum* BF2, *Bifidus lactis* BL2, *Bifidobacterium infantis* BT1 und *Streptococcus thermophilus* ST3.

4. Zusammensetzung nach einem der vorhergehenden Ansprüche, **dadurch gekennzeichnet, dass** die Mikroorganismen mit mindestens einem Protein und mindestens einem Polysaccharid beschichtet, insbesondere doppelt beschichtet, vorliegen.

5. Zusammensetzung nach einem der vorhergehenden Ansprüche umfassend mindestens eine prebiotische Verbindung ausgewählt aus einer Gruppe enthaltend Fructooligosaccharide. Inulin, Pektin, Glucane, Galaktane, Dextrose, Oligosaccharide wie Galactooligosaccharide, Sojaoligosaccharide, Raffinosesaccharide, Stachyose, Bananenfaser, Mannane, Guarkernmehl, Konjakmehl, Xanthan, Pentosan, beta-Glucan, Arabinan, Galactan, Arabinogalactan und Mischungen aus diesen.

6. Zusammensetzung nach einem der vorhergehenden Ansprüche, ferner umfassend mindestens einen Hüfsstoff ausgewählt aus der Gruppe enthaltend einen Füllstoff, einen Trägerstoff, ein Trennmittel, ein Streckmittel, ein Bindemittel, ein Netzmittel, ein Stabilisierungsmittel, ein Färbemittel, einen Pufferstoff, einen Riechstoff und einen Geschmacksstoff.

7. Zusammensetzung nach Anspruch 6, **dadurch gekennzeichnet, dass** der mindestens einen Füllstoff ausgewählt ist aus einer Gruppe enthaltend Cellulose, Lactose, Saccharose, Amylose, Dextrose, Mannit, Inosit, Maltodextrin, Maisstärke, Reisstärke, Ballaststoffe, Calciumhydrogenphosphate und Hydrokolloide.

8. Zusammensetzung nach Anspruch 6, **dadurch gekennzeichnet, dass** das mindestens eine Trennmittel ausgewählt ist aus einer Gruppe enthaltend Siliziumdioxid, Talkum, Magnesium- oder Calciumcarbonat, Cellulose bzw. mikrokristalline Cellulose, Magnesiumcarbonat, Magnesiumsalze von Speisefettsäuren, kolloidale Kieselsäure, , Natrium-, Magnesium-, Calcium- und Aluminiumsilikate, Bentonit, Dicalciumdiphosphat, Calciumnatriumpolyphoshat, Calciumpolyphosphate und Stearinsäure.

9. Zusammensetzung nach Anspruch 6, **dadurch gekennzeichnet, dass** der mindestens eine Trägerstoff ausgewählt ist aus einer Gruppe enthaltend Lecithine, Alginate, Traganth, Gummi arabicum, Glucitol, Pektine, Carboxymethylcellulose, Saccharose, Milchzucker und Stärke.

10. Zusammensetzung nach einem der vorhergehenden Ansprüche, **gekennzeichnet durch** folgende quantitative Zusammensetzung:
a. 1 bis 20 Gew% einer Mischung aus mindestens zwölf probiotischen Mikroorganismen umfassend mindestens 6 Lactobacillusstämme, mindestens 5 Bifidusbakterienstämme und mindestens einen Stamm aus der Gruppe der Streptokokken, bevorzugt 2 bis 15 Gew%, insbesondere bevorzugt 4 bis 10 Gew%
b. 10 bis 60 Gew% mindestens einer präbiotischen Substanz, bevorzugt 20 bis 50 Gew%, insbesondere bevorzugt 30 bis 40 Gew%, und
c. 0 bis 70 Gew% mindestens eines Hilfsstoffes.

11. Zusammensetzung nach einem der vorhergehenden Ansprüche, **gekennzeichnet durch** eine Lagerstabilität bei 20°C von mindestens 7 Wochen, bevorzugt von 9 Wochen, insbesondere bevorzugt von 11 Wochen.

12. Zusammensetzung nach einem der vorhergehenden Ansprüche zur Verwendung als Nahrungsergänzungsmittel zur Vorbeugung und Behandlung von allergischen Reaktionen, insbesondere Neurodermitis, Asthma und/oder Rhinitis.

## Claims

1. Composition comprising at least twelve probiotic microorganisms, wherein the composition comprises at least 6 lactobacillus strains, at least 5 Bifidus bacteria strains and at least one strain from the group of streptococcus.

2. Composition according to claim 1, **characterized in that** the microorganisms are selected from a group containing *Lactobacillus acidophilus* LH5, *Lactobacillus casei* LC1, *Lactobacillus plantarum* LP1, *Lactobacillus rhamnosus* LR3, *Lactococcus lactis* SL6, *Lactobacillus paracasei* LPC, *Lactobacillus bulgaricus* LG1, *Bifidus longum* BG3, *Bifidus breve* BR2, *Bifidus bifidum* BF2, *Bifidus lactis* BL2, *Bifidus faecium, Bifidus faecalis, Bifidobacterium infantis* BT1, *Streptococcus thermophilus* ST3, *Lactobacillus buchneri, L. brevis, L. cellobiosus, L. collinoides, L. coprophillus, L. crispatus, L. curvatus, L. delbrückkii ssp. delbrückkii, L. delbrückkii ssp. bulgaricus, L.delbrückkii ssp. lactis, L. fermentum, L.fructiforans, L. gasseri, L. helveticus, L. johnsonii, L. lindneri, ,L. reuterii, L. periosus, L.salivarius, Bifidus adolescens* and *B. animalis*.

3. Composition according to claim 1 or 2, **characterized in that** the microorganisms are selected from a group containing *Lactobacillus acidophilus* LH5, *Lactobacillus casei* LC1, *Lactobacillus plantarum* LP1, *Lactobacillus rhamnosus* LR3, *Lactobacillus paracasei* LPC, *Lactobacillus bulgaricus* LG1, *Bifidus longum* BG3, *Bifidus breve* BR2, *Bifidus bifidum* BF2, *Bifidus lactis* BL2, *Bifidobacterium infantis* BT1 and *Streptococcus thermophilus* ST3.

4. Composition according to one of the preceding claims, **characterized in that** the microorganisms are coated with at least on protein and at least one polysaccharide, in particular double coated.

5. Composition according to one of the preceding claims, comprising at least one pre-biotic compound selected from a group containing fructooligosaccharides, inulin, pectin, glucanes, galactanes, dextrose, oligosaccharides such as galactooligosaccharides, soy oligosaccharides, raffinose saccharides, stachyose, banana fiber, mannanes, guar gum, conjac flower, xanthane, pentosan, beta-glucane, arabinane, galactane, arabino galactane and mixtures thereof.

6. Composition according to one of the preceding claims furthermore comprising at least one auxiliary agent selected from the group containing a filler, a carrier, separating agent, an extender, a binding agent, a wetting agent, a stabilizer, a colouring agent, a buffer, an aromatic substance and a flavouring agent.

7. Composition according to claim 6, **characterized in that** the at least one filler is selected from a group containing cellulose, lactose, saccarose, amylase, dextrose, mannitol, inositol, maltodextrin, corn starch, rice starch, fibers, calciumhydrogenphosphate and hydrocolloides.

8. Composition according to claim 6, **characterized in that** that the at least one separating agent is selected from a group containing silicium dioxide, talcum, magnesium or calcium carbonate, cellulose or micro crystalline cellulose, magnesium carbonate, magnesium salts of cooking fatty acids, colloidal silicic acid, sodium, magnesium, calcium and aluminium silicates, bentonite, dicalciumdiphosphate, calcium sodium polyphosphate, calcium polyphosphate and stearic acid.

9. Composition according to claim 6, **characterized in that** the at least one carrier is selected from a group containing lecithines, alginates, traganth, gum arabicum, glucitol, pectines, carboxymethylcellulose, saccharose, milk sugar and starch.

10. Composition according to one of the preceding claims, **characterized by** the following quantitative composition:
a. 1 to 20 weight% of a mixture of at least twelve probiotic microorganisms comprising at least 6 lactobacillus strains, at least 5 Bifidus bacteria strains and at least one strain from the group of the streptococcus, preferably 2 to 15 weight %, in particular preferably 4 to 10 weight%,
b. 10 to 60 weight% of at least one pre-biotic substance, preferably 20 to 50 weight%, in particular preferably 30 to 40 weight%, and
c. 0 to 70 weight% of at least one auxiliary agent.

11. Composition according to one of the preceding claims, **characterized by** a storing stability at 20°C of at least 7 weeks, preferably of 9 weeks, in particular preferably of 11 weeks.

12. Composition according to one of the preceding claims for use as a food supplement for preventing and treating allergic reactions, in particular, neurodermatitis, asthma and/or rhinitis.

## Revendications

1. Composition comprenant au moins douze micro-organismes probiotiques, où la composition comprend au moins 6 souches de Lactobacillus, au moins 5 souches de bactéries Bifidus et au moins une souche du groupe des streptocoques.

2. Composition selon la revendication 1, **caractérisée en ce que** les micro-organismes sont choisis dans un groupe contenant *Lactobacillus acidophilus* LH5, *Lactobacillus casei* LC1, *Lactobacillus plantarum* LP1, *Lactobacillus rhamnosus* LR3, *Lactococcus lactis* SL6, *Lactobacillus paracasei* LPC, *Lactobacillus bulgaricus* LG1, *Bifidus longum* BG3, *Bifidus breve* BR2, *Bifidus bifidum* BF2, *Bifidus lactis* BL2, *Bifidus faecium, Bifidus faecalis, Bifidobacterium infantis* BT1, *Streptococcus thermophilus* ST3, *Lactobacillus buchneri, L. brevis, L. cellobiosus, L. collinoides, L. coprophillus, L. crispatus, L. curvatus, L. delbrückkii ssp. delbrückkii, L. delbrückkii ssp. bulgaricus, L. delbrückkii ssp. lactis, L. fermentum, L. fructiforans, L. gasseri, L. helveticus, L. johnsonii, L. lindneri, L. reuterii, L. periosus, L. salivarius, Bifidus adolescens* et *B. animalis.*

3. Composition selon la revendication 1 ou 2, **caractérisée en ce que** les micro-organismes sont choisis dans un groupe contenant *Lactobacillus acidophilus* LH5, *Lactobacillus casei* LC1, *Lactobacillus plantarum* LP1, *Lactobacillus rhamnosus* LR3, *Lactobacillus paracasei* LPC, *Lactobacillus bulgaricus* LG1, *Bifidus longum* BG3, *Bifidus breve* BR2, *Bifidus bifidum* BF2, *Bifidus lactis* BL2, *Bifidobacterium infantis* BT1 et *Streptococcus thermophilus* ST3.

4. Composition selon l'une des revendications précédentes, **caractérisée en ce que** les micro-organismes sont présents recouverts, en particulier doublement recouverts, d'au moins une protéine et d'au moins un polysaccharide.

5. Composition selon l'une des revendications précédentes comprenant au moins un composé prébiotique choisi dans un groupe contenant les fructooligosaccharides, l'inuline, la pectine, les glucanes, les galactanes, le dextrose, des oligosaccharides comme les galactooligosaccharides, les oligosaccharides du soja, les saccharides du raffinose, le stachyose, les fibres de la banane, le mannane, la farine de graines de guar, la farine de conjac, le xanthane, le pentosane, le bêta-glucane, l'arabinane, le galactane, l'arabinogalactane et les mélanges de ceux-ci.

6. Composition selon l'une des revendications précédentes, comprenant en outre au moins un adjuvant choisi dans le groupe contenant une charge, un vecteur, un agent de séparation, un agent d'étalement, un liant, un humectant, un stabilisant, un colorant, un tampon, un arôme et un agent de sapidité.

7. Composition selon la revendication 6, **caractérisée en ce que** la au moins une charge est choisie dans un groupe contenant la cellulose, le lactose, le saccharose, l'amylose, le dextrose, le mannitol, l'inositol, la maltodextrine, l'amidon de maïs, l'amidon de riz, les substances ballast, les hydrogénophosphates de calcium et les hydrocolloïdes.

8. Composition selon la revendication 6, **caractérisée en ce que** le au moins un agent de séparation est choisi dans un groupe contenant le dioxyde de silicium, le talc, le carbonate de magnésium ou de calcium, la cellulose et la cellulose microcristalline, le carbonate de magnésium, les sels de magnésium et d'acides gras alimentaires, l'acide silicique colloïdal, le dioxyde de silicium, les silicates de sodium, de magnésium, de calcium et d'aluminium, la bentonite, le diphosphate de dicalcium, le polyphosphate de calcium et sodium, les polyphosphates de calcium et l'acide stéarique.

9. Composition selon la revendication 6, **caractérisée en ce que** le au moins un vecteur est choisi dans un groupe contenant les lécithines, les alginates, la gomme adragante, la gomme arabique, le glucitol, les pectines, la carboxyméthylcellulose, le saccharose, le lactose et l'amidon.

10. Composition selon l'une des revendications précédentes, **caractérisée par** la composition quantitative suivante:
a. 1 à 20 % en poids d'un mélange d'au moins douze micro-organismes probiotiques comprenant au moins 6 souches de Lactobacillus, au moins 5 souches de bactéries Bifidus et au moins une souche du groupe des streptocoques, de préférence 2 à 15 % en poids, de manière particulièrement préférée 4 à 10 % en poids
b. 10 à 60 % en poids d'au moins une substance prébiotique, de préférence 20 à 50 % en poids, de manière particulièrement préférée 30 à 40 % en poids, et
c. 0 à 70 % en poids d'au moins un adjuvant.

11. Composition selon l'une des revendications précédentes **caractérisée par** une stabilité au stockage à 20°C d'au moins 7 semaines, de préférence de 9 semaines, de manière particulièrement préférée de 11 semaines.

12. Composition selon l'une des revendications précédentes destinée à être utilisée comme complément alimentaire pour la prévention et le traitement des réactions allergiques, en particulier de la neurodermite, de l'asthme et/ou de la rhinite.
